# EUROPÄISCHE PATENTSCHRIFT

(11) **EP 2 626 304 B1**
(45) Veröffentlichungstag und Bekanntmachung des Hinweises auf die Patenterteilung: **25.06.2014**
(21) Anmeldenummer: 12000863.6
(22) Anmeldetag: 10.02.2012
(51) Int. Cl.: B65B 3/00, B65B 7/26, B65B 43/54, B65B 51/14, A61J 3/07, A61M 15/00

(54) **Füll- und Verschliesseinrichtung sowie Verfahren zum Füllen und Verschliessen eines Wirkstoffapplikators**
Filling and closing device and method for filling and closing an agent applicator
Dispositif de remplissage et de fermeture ainsi que procédé de remplissage et de fermeture d'un applicateur de matière active

(43) Veröffentlichungstag der Anmeldung: 14.08.2013
(73) Patentinhaber: Harro Höfliger Verpackungsmaschinen GmbH, 71573 Allmersbach im Tal (DE)
(72) Erfinder: Joos, Thomas, 74417 Gschwend (DE); Götz, Achim, 71364 Winnenden (DE); Bohn, Markus, 70567 Stuttgart (DE); Mattern, Udo, 6376 Emmetten (CH); Mattern, Claudia, Dr., 6376 Emmetten (CH)
(74) Vertreter: Zurhorst, Stefan

(56) Entgegenhaltungen:
- EP-A1- 2 048 083
- US-A- 5 215 221

## Beschreibung

Die Erfindung betrifft eine Füll- und Verschließeinrichtung sowie ein Verfahren zum Füllen und Verschließen eines Wirkstoffapplikators.

Bei bestimmten Wirkstoffpräparaten für den menschlichen Körper kommt es auf eine exakte Dosierung an. Um eine solche exakte Dosierung durch den Benutzer zu gewährleisten, werden Dosiereinheiten bereitgestellt, die die vorgesehene Menge des Wirkstoffpräparates enthalten, und die bei der Anwendung genau diese vorgesehene Menge dem menschlichen Körper zuführen. Darüber hinaus sind auch Wirkstoffapplikatoren bekannt, die für die einmalige Benutzung vorgesehen sind und dabei eine Doppelfunktion ausüben: Zum einen enthalten sie eine fest vorgegebene Dosiermenge des Wirkstoffpräparates. Zum anderen dienen sie nicht nur der Bereitstellung, sondern auch der Zufuhr des Wirkstoffpräparates in der gewünschten Menge an die gewünschte Stelle. Nach erfolgter Anwendung wird die entleerte Einheit entsorgt. Ein solcher Wirkstoffapplikator für die Bereitstellung und Verabreichung einer Einheitsdosis in die Nase ist aus dem eingetragenen Gemeinschaftsgeschmacksmuster 001790908-0001 bekannt.

Der genannte Wirkstoffapplikator weist ein Unterteil und ein Deckelteil auf, zwischen denen ein Aufnahmeraum für ein Wirkstoffpräparat ausgebildet ist. Das Unterteil und das Deckelteil weisen je einen umlaufenden Siegelrand zur dichtenden Verbindung des Deckelteils mit dem Unterteil auf. Das Unterteil ist mit einer Applikationsröhre versehen, die in den Aufnahmeraum mittels einer inneren Mündung mündet. Bezogen auf die Ebene der gefügten Siegelränder ragt die für die Einführung in die Nase vorgesehene Applikationsröhre in einem flachen Winkel ausgehend vom Aufnahmeraum über die Siegelränder hervor und deckt dadurch einen Stützflächenabschnitt des Unterteil-Siegelrandes ab.

Ein Kunststoffrohling zur Bildung des Wirkstoffapplikators kann als Spritzgussteil kostengünstig unter den Bedingungen einer Großserienfertigung hergestellt werden. Das Befüllen und Verschließen dieses Kunststoffrohlings gestaltet sich jedoch schwierig wegen der geometrischen Anordnung der Applikationsröhre. Für die Befüllung ist eine lagegenaue Positionierung des noch offenen Rohlings erforderlich, bei der die Applikationsröhre im Wege ist. Für den anschließenden Verschließvorgang, bei dem das Deckelteil auf das Unterteil mit der Applikationsröhre aufgesiegelt wird, muss am Siegelrand des Unterteils ein Gegendruck aufgebracht werden, der sich über die gesamte Siegelfläche erstreckt. Nur so kann eine ringsum wirkende, hermetisch verschlossene Versiegelung sichergestellt werden. Im Bereich der Applikationsröhre ist aber der Siegelrand durch diese abgedeckt, so dass hier der Zugriff auf den Siegelrand zum Aufbringen des Gegendrucks verwehrt ist.

Der Erfindung liegt die Aufgabe zugrunde, eine Füll- und Verschließeinrichtung für den oben genannten Wirkstoffapplikator anzugeben, mittels dessen eine lagegenaue Positionierung, eine Befüllung, eine zuverlässig dichte Versiegelung und eine automatisierte Entnahme des fertigen Wirkstoffapplikators möglich ist.

Diese Aufgabe wird durch eine Füll- und Verschließeinrichtung mit den Merkmalen des Anspruchs 1 gelöst.

Der Erfindung liegt des Weiteren die Aufgabe zugrunde, ein Verfahren zum Füllen und Verschließen des genannten Wirkstoffapplikators anzugeben, welches bei hohen Taktzahlen eine zuverlässig dichte Versiegelung sicherstellt.

Diese Aufgabe wird durch ein Verfahren mit den Merkmalen des Anspruchs 7 gelöst.

Nach der erfindungsgemäßen Lösung weist die Füll- und Verschließeinrichtung ein Aufnahmeelement für das Unterteil des Wirkstoffapplikators auf. Das Aufnahmeelement ist mit einer umlaufenden Druckfläche zur Anlage der Stützfläche des Unterteils sowie mit einem unter die Druckfläche reichenden Aufnahmekanal für die Applikationsröhre des Unterteils versehen. Das Aufnahmeelement ist mindestens zweiteilig mit einem Basiselement und einem gegenüber dem Basiselement beweglichen Abdeckelement aufgebaut. Der Aufnahmekanal ist zwischen dem Basiselement und dem Abdeckelement ausgebildet. Die umlaufende Druckfläche erstreckt sich über das Basiselement und das Abdeckelement derart, dass auf der dem Aufnahmekanal gegenüberliegenden Oberseite des Abdeckelements ein Druckflächenabschnitt zur Anlage des durch die Applikationsröhre verdeckten Stützflächenabschnittes des Wirkstoffapplikators ausgebildet ist.

Im zugehörigen Verfahren wird das Unterteil des Wirkstoffapplikators derart in das Aufnahmeelement eingelegt, dass die Applikationsröhre in den Aufnahmekanal hineinragt. Die umlaufende Stützfläche des Unterteils liegt dabei auf der umlaufenden Druckfläche derart auf, dass auch der verdeckte Stützflächenabschnitt auf dem Druckflächenabschnitt des Abdeckelementes aufliegt. In diesem Zustand ist eine exakte Lagefixierung des Unterteils im Aufnahmeelement dadurch sichergestellt, dass einerseits die Applikationsröhre in den Aufnahmekanal hineinragt und dort gehalten wird. Andererseits kann das bewegliche Abdeckelement in die Hinterschneidung zwischen der Applikationsröhre und dem Siegelrand des Unterteils gebracht werden, so dass trotz dieser Hinterschneidung auch im verdeckten Stützflächenabschnitt eine Stützwirkung durch den Druckflächenabschnitt des Abdeckelementes eintritt. Insgesamt ist dadurch der gesamte Siegelrand des Applikatorunterteils entlang der gesamten umlaufenden Stützfläche abgestützt, was zur exakten Lagefixierung beträgt. Im nachfolgenden Verfahrensschritt wird eine Aufnahmeraumausformung des in vorgenannter Weise exakt fixierten Unterteils mit dem Wirkstoffpräparat befüllt.

Nach der Befüllung wird das Oberteil des Wirkstoffapplikators lagerichtig derart auf das Unterteil gebracht, dass die umlaufenden Siegelränder aufeinanderliegen. Daran anschließend wird das Oberteil auf das Unterteil an den umlaufenden Siegelrändern unter Gegendruck der umlaufenden Druckfläche aufgesiegelt. Hier kommt insbesondere das bewegliche Abdeckelement zum Tragen, welches in vorgenannter Weise in die Hinterschneidung zwischen der Applikationsröhre und dem hierdurch verdeckten Stützflächenabschnitt hineinragt und folglich auch an dieser Stelle - trotz der darunter liegenden Applikationsröhre - einen Gegendruck gegen den von oben einwirkenden Siegeldruck ausüben kann. Durch die zweiteilige, bewegliche Bauform kann also trotz des Vorhandenseins der Applikationsröhre eine vollständig geschlossen umlaufende Druckfläche bereitgestellt werden, die eine unterbrechungsfreie umlaufende dichte Siegelnaht erzeugt.

Hieran anschließend wird das Abdeckelement gegenüber dem Basiselement derart bewegt, dass der zwischen ihnen ausgebildete Aufnahmekanal nach oben freiliegt. Der nun nach oben offene Aufnahmekanal erlaubt es, dass der fertig befüllte und versiegelte Wirkstoffapplikator mittels eines Greifers automatisiert nach oben angehoben und abgeführt wird.

In einer bevorzugten Weiterbildung der Füll- und Verschließeinrichtung ist am Wirkstoffapplikator bezogen auf seinen Längsschnitt zwischen dem verdeckten Stützflächenabschnitt und der Applikationsröhre eine konkave Kante ausgebildet, wobei am beweglichen Abdeckelement eine Andruckkante zur Anlage an der genannten konkaven Kante des Wirkstoffapplikators ausgebildet ist. Über seine Stütz- und Haltewirkung in senkrechter Richtung hinausgehend ist durch die Wechselwirkung zwischen der Andruckkante des Abdeckelements und der konkaven Kante des Wirkstoffapplikators auch eine horizontal bzw. lateral wirkende formschlüssige Lagesicherung des Wirkstoffapplikators gegenüber dem Aufnahmeelement herbeigeführt.

In zweckmäßiger Weiterbildung geht die vorgenannte Andruckkante des Abdeckelementes in eine Haltefläche zur Anlage an zumindest einem Abschnitt der Applikationsröhre des Wirkstoffapplikators über. Während die vorgenannte umlaufende Druckfläche allein das Gewicht des Applikatorrohlings und ggf. auch den Siegeldruck trägt, wirkt die zusätzliche Haltefläche in Gegenrichtung, so dass der Applikatorrohling nicht ohne weiteres eine von der Druckfläche abhebende Aufwärtsbewegung ausüben kann. Beschleunigungen und Verzögerungen im Laufe des gesamten Füll- und Verschließprozesses können nicht dazu führen, dass der Applikatorrohling bzw. der fertig befüllte Applikator aus seiner Aufnahme herausspringt.

In einer bevorzugten Ausführungsform ist im Basiselement innerhalb der umlaufenden Druckfläche eine Aufnahme für die Aufnahmeraumausformung des Unterteils des Wirkstoffapplikators ausgebildet. Einerseits stellt dieser sicher, dass das Applikator-Unterteil tatsächlich mit seinem Siegelrand bzw. seiner umlaufenden Stützfläche auf der umlaufenden Druckfläche des Aufnahmeelementes aufliegen kann, ohne von der Aufnahmeraumausformung abgehoben zu werden. Andererseits trägt die Aufnahme bei einer geometrischen Anpassung an die Aufnahmeraumausformung dazu bei, dass das Unterteil des Applikator-Rohlings in horizontaler bzw. lateraler Richtung formschlüssig lagegenau fixiert ist.

Es kann zweckmäßig sein, das Abdeckelement gegenüber dem Basiselement beispielsweise linear verschieblich auszugestalten. In bevorzugter Weiterbildung ist das Abdeckelement gegenüber dem Basiselement um eine Schwenkachse schwenkbar, wobei die Schwenkachse senkrecht auf der umlaufenden Druckfläche steht. Nach erfolgter Befüllung und Versiegelung kann das Abdeckelement gegenüber dem Basiselement um die genannte Schwenkachse geschwenkt werden, ohne dass es dabei zu einem Höhenversatz gegenüber der Druckfläche kommt. Der Wirkstoffapplikator behält während des Schwenkvorganges seine zuvor vorhandene Position bei und kann leicht von einem automatisierten Greifer aufgenommen und vertikal angehoben werden, bis die angeformte Applikationsröhre vollständig aus ihrem Aufnahmekanal herausgehoben ist. Der Wirkstoffapplikator steht dann nicht mehr in Wechselwirkung mit dem Aufnahmeelement und kann mittels des Greifers beliebig abgeführt werden.

Ein Ausführungsbeispiel der Erfindung ist nachfolgend anhand der Zeichnung näher beschrieben. Es zeigen:
- Fig. 1: in einer perspektivischen Unteransicht einen Wirkstoffapplikator für die Nase mit einer hervorstehenden Applikationsröhre;
- Fig. 2: einen aufgeklappten Kunststoffrohling zur Bildung des Wirkstoffapplikators nach Fig. 1 mit einem Unterteil und mit einem Deckelteil zur Darstellung geometrischer Einzelheiten;
- Fig. 3: eine perspektivische Oberansicht des aus dem Kunststoffrohling nach Fig. 2 gebildeten Wirkstoffapplikators mit gegenüber dem Unterteil zugeklapptem und verrastetem Deckelteil;
- Fig. 4: in einer Draufsicht eine Übersichtsdarstellung der erfindungsgemäßen Füll- und Verschließeinrichtung für den Wirkstoffapplikator nach den Fig. 1 bis 3 mit seinen verschiedenen, für das erfindungsgemäße Verfahren vorgesehenen Stationen;
- Fig. 5: eine perspektivische Detailansicht der Füll- und Verschließanordnung nach Fig. 4 im Bereich ihrer Einlegestation;
- Fig. 6: eine perspektivische Detailansicht der Füll- und Verschließeinrichtung nach Fig. 4 mit Einzelheiten ihrer Siegelstation;
- Fig. 7: eine perspektivische Detailansicht der Füll- und Verschließeinrichtung nach Fig. 4 im Bereich ihrer Übergabestation;
- Fig. 8: eine Längsschnittdarstellung eines Aufnahmeelements der Füll- und Schließeinrichtung nach den Fig. 4 bis 7 mit eingelegtem Unterteil des Wirkstoffapplikators nach den Fig. 1 bis 3;
- Fig. 9: eine vergrößerte Darstellung der Einzelheit IX nach Fig. 8 mit Details der Wechselwirkung zwischen einem Abdeckelement des Aufnahmeelementes und dem Unterteil des Wirkstoffapplikators im Bereich seiner Applikationsröhre;
- Fig. 10: die Anordnung nach Fig. 8 beim Aufsiegeln des Deckelteils auf das Unterteil des Wirkstoffapplikators;
- Fig. 11: die Anordnung nach den Fig. 8 bis 10 mit aufgeschwenktem Abdeckelement bei der vertikalen Entnahme des fertig befüllten und versiegelten Wirkstoffapplikators.

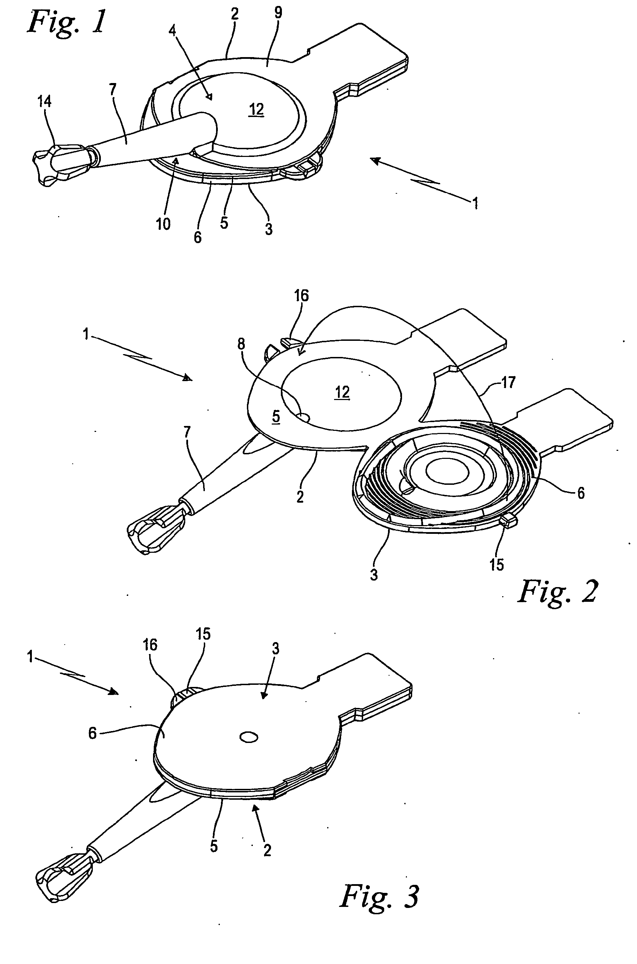

Fig. 1 zeigt in einer perspektivischen Unteransicht einen Wirkstoffapplikator 1 zur Aufbewahrung einer Einheitsdosis eines Wirkstoffpräparates und zur Verabreichung dieser Einheitsdosis in die menschliche Nase. Der Wirkstoffapplikator 1 umfasst ein Unterteil 2 sowie ein Deckelteil 3, zwischen denen ein Aufnahmeraum 4 für das genannte Wirkstoffpräparat ausgebildet ist. Zur Bildung des Aufnahmeraums 4 weist das Unterteil 2 eine von einem Siegelrand 5 umschlossene Aufnahmeraumausformung 12 auf, die im gezeigten Ausführungsbeispiel als Kugelabschnitt ausgebildet ist. Ausgehend von der Aufnahmeraumausformung 12 erstreckt sich eine einteilig angeformte Applikationsröhre 7, die an ihrem freien Ende mit einem ebenfalls einteilig angeformten Verschluss 14 verschlossen ist. Für die Anwendung wird zunächst der Verschluss 14 abgebrochen bzw. abgedreht und entfernt. Anschließend wird die Applikationsröhre 7 in ein Nasenloch eingeführt, wobei dann durch Finger- bzw. Daumendruck auf die Aufnahmeraumausformung 12 das im Aufnahmeraum 4 vorgehaltene Wirkstoffpräparat durch die Applikationsröhre 7 in die Nase gedrückt wird.

Das Unterteil 2 und das Deckelteil 3 weisen je einen um den Aufnahmeraum 4 umlaufenden Siegelrand 5, 6 auf, durch den sie dichtend miteinander verbunden bzw. versiegelt sind. Am Unterteil 2 ist einteilig die Applikationsröhre 7 angeformt, die sich ausgehend von einer Aufnahmeraumausformung 12 des Unterteils 2 in einem spitzen Winkel bezogen zur Ebene der Siegelränder 5, 6 erstreckt und dabei den Siegelrand 5 des Unterteils 2 mit Abstand zumindest teilweise - hier vollständig übergreift. Der Siegelrand 5 des Unterteils bildet auf seiner dem Deckelteil 3 abgewandten Unterseite, welche in der zeichnerischen Darstellung nach Fig. 1 oben liegt, eine umlaufende Stützfläche 9. Da die Applikationsröhre 7 den Siegelrand 5 und dessen umlaufende Stützfläche 9 mit Abstand übergreift, ist zwischen der Applikationsröhre 7 und dem Siegelrand 5 ein durch die Applikationsröhre 7 nach Art einer Hinterschneidung zumindest teilweise verdeckter Stützflächenabschnitt 10 der Stützfläche 9 gebildet.

Fig. 2 zeigt in einer perspektivischen Ansicht einen Kunststoffrohling zur Bildung des Wirkstoffapplikators 1 nach Fig. 1, wobei in dem gezeigten Kunststoffrohling das Unterteil 2 mit der Applikationsröhre 7 und das Deckelteil 3 einteilig hergestellt und miteinander verbunden sind. Es ist aber auch eine zweiteilige Ausführung mit dem Unterteil 2 und dem davon getrennten Deckelteil 3 möglich. In jedem Falle ist in der Aufnahmeraumausformung 12 eine Mündung 8 ausgebildet, mittels derer die Applikationsröhre 7 in den Aufnahmeraum 4 (Fig. 1) mündet.

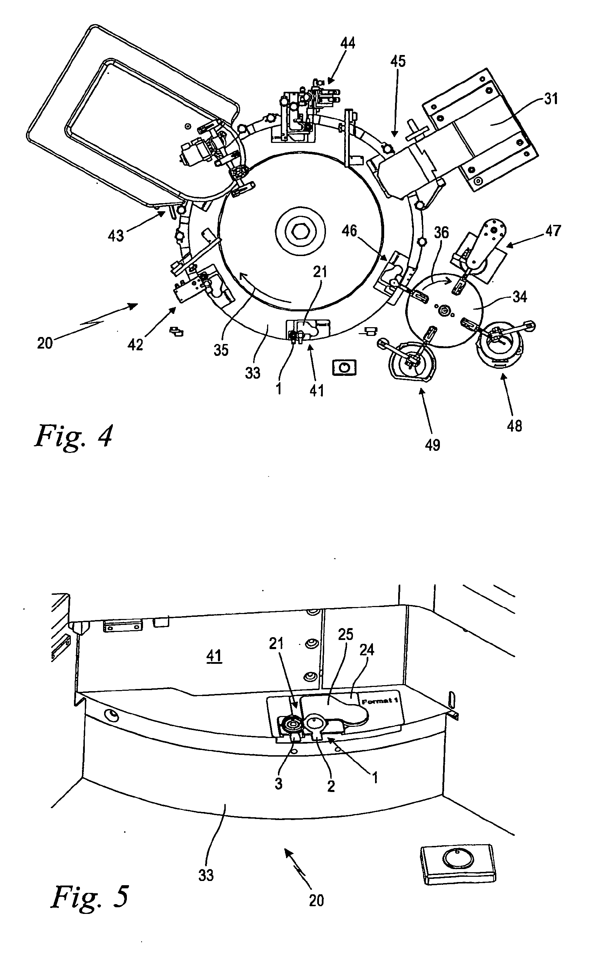

Für die Befüllung und für das Verschließen des Wirkstoffapplikators 1 wird der Rohling in seiner Position nach Fig. 2 in die Füll- und Verschließeinrichtung 20 eingelegt, welche nachfolgend im Zusammenhang mit den Fig. 4 bis 11 beschrieben ist. Die Aufnahmeraumausformung 12 ist dabei bezogen auf die Gewichtskraftsrichtung nach oben offen und kann mit dem Wirkstoffpräparat befüllt werden. Nach erfolgter Befüllung wird das Deckelteil 3 entsprechend einem Pfeil 17 derart auf das Unterteil 2 geklappt, dass sein Siegelrand 6 auf dem um die Aufnahmeraumausformung 12 umlaufenden Siegelrand 5 zu liegen kommt, wie dies in der perspektivischen Oberansicht des Wirkstoffapplikators 1 nach Fig. 3 dargestellt ist. Aus der Zusammenschau der Fig. 2 und 3 ergibt sich, dass beim Zusammenklappen eine am Deckelteil 3 angeformte Rastnase 15 in eine korrespondierende Rastaufnahme 16 des Unterteils 2 einrastet und hierdurch das Deckelteil 3 entsprechend der Darstellung nach Fig. 3 in seiner provisorisch geschlossenen Position gegenüber dem Unterteil 2 hält. Der befüllte und provisorisch geschlossene Wirkstoffapplikator 1 ist in der Darstellung nach Fig. 3 fertig vorbereitet für die Versiegelung, wie sie weiter unten im Zusammenhang mit der Füll- und Verschließeinrichtung 20 nach den Fig. 4 bis 11 beschrieben ist.

Fig. 4 zeigt in einer Draufsicht eine Übersichtsdarstellung einer erfindungsgemäßen Füll- und Verschließreinrichtung 20. Die Füll- und Verschließeinrichtung 20 umfasst einen ersten, größeren Rundlauftisch 33, der im Betrieb drehend entsprechend einem Pfeil 35 bewegbar ist, sowie einen zweiten, im Vergleich dazu kleineren Rundlauftisch 34, der im Betrieb drehend entsprechend einem Pfeil 36 bewegbar ist. Die erfindungsgemäße Füll-und Verschließeinrichtung 20 ist zum Füllen und Verschließen des Wirkstoffapplikators 1 nach den Fig. 1 bis 3 bzw. des dort dargestellten Kunststoffrohlings vorgesehen und bildet gemeinsam damit ein gegenseitig aufeinander abgestimmtes System.

Mit den beiden Rundlauftischen 33, 34 wird der Wirkstoffapplikator 1 nach den Fig. 1 bis 3 insgesamt neun verschiedenen Bearbeitungsstationen zugeführt. Der erste Rundlauftisch 33 ist hierzu mit insgesamt sechs über seinen Umfang verteilten Aufnahmeleementen 21 versehen, mittels derer je ein Wirkstoffapplikator 1 bzw. je ein Rohling davon den ersten sechs Bearbeitungsstationen zuführbar ist. Eine erste Station 41 dient der Einlage des Wirkstoffapplikators 1 bzw. seines unbefüllten und noch geöffneten Kunststoffrohlings. Nach erfolgter Einlage wird dieser einer zweiten Station 42 für die Anwesenheits- und Höhenkontrolle zugeführt. Danach wird eine dritte Station 43 zur Befüllung der Aufnahmeraumausformung 12 nach Fig. 2 angefahren. Dieser dritten Station 43 folgt eine vierte Station 44, in der das Deckelteil 3 auf das Unterteil 2 entsprechend der Darstellung nach den Fig. 2 und 3 geklappt wird, um ein provisorisches Verschließen herbeizuführen. In einer nachfolgenden fünften Station 45 ist eine Siegelvorrichtung 31 angeordnet, mittels derer das Deckelteil 3 auf das Unterteil 2 (Fig. 1 bis 3) aufgesiegelt wird.

In einer nachfolgenden sechsten Station 46 erfolgt die Übergabe des befüllten und versiegelten Wirkstoffapplikators 1 zum zweiten Rundlauftisch 34, mittels dessen eine siebte Station 47 zur Dichtigkeitsprüfung des Wirkstoffapplikators 1 angefahren wird. In einer achten Station 48 erfolgt eine Schlechtteileausscheidung, während in der nachfolgenden, letzten und neunten Station 49 die Entnahme der Gutteile, also der korrekt befüllten und verschlossenen Wirkstoffapplikatoren 1 erfolgt.

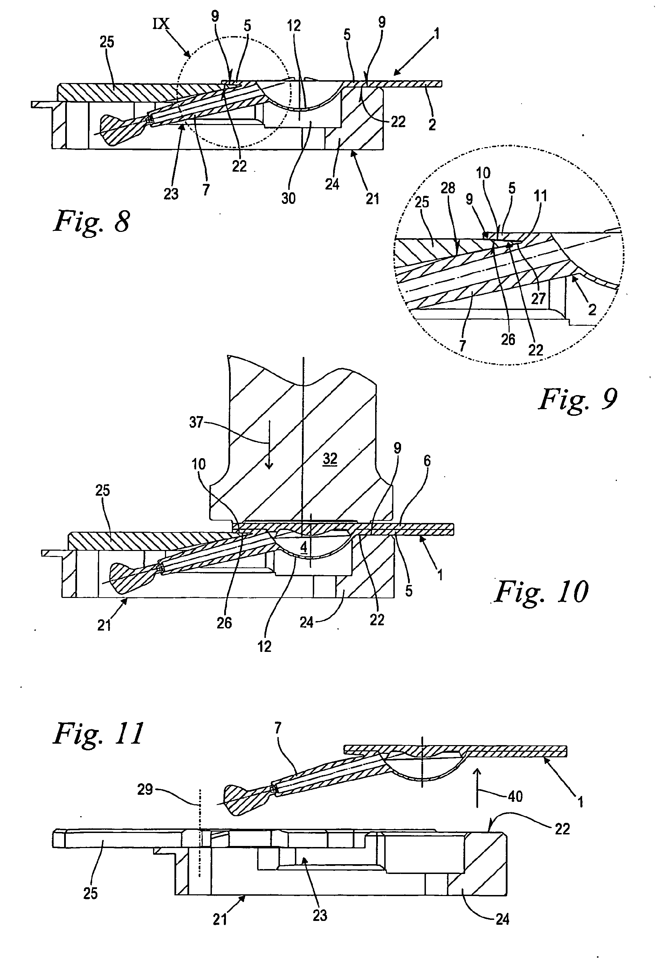

Fig. 5 zeigt in einer perspektivischen Detailansicht die Füll- und Verschließeinrichtung 20 nach Fig. 4 im Bereich ihrer ersten Station 41. Das hier gezeigte eine von insgesamt sechs Aufnahmeelementen 21 am ersten Rundlauftisch 33 ist mindestens zweiteilig ausgeführt und für die Aufnahme des geöffneten Rohlings des Wirkstoffapplikators 1 vorgesehen, wobei das Aufnahmeelment 21 als erstes Teil ein Basiselement 24 und als zweites Teil ein gegenüber dem Basiselement 24 bewegliches Abdeckelement 25 umfasst. Einzelheiten zur Wirkungsweise und gegenseitiger Interaktion zwischen dem Basiselement 24, dem Abdeckelement 25 und dem Wirkstoffapplikator 1 sind weiter unten im Zusammenhang mit den Fig. 8 bis 11 näher beschrieben.

In der ersten Station 41 wird der geöffnete Kunststoffrohling des Wirkstoffapplikators 1 in seiner Konfiguration nach Fig. 2 in das Aufnahmeelement 21 derart eingelegt, dass das Unterteil 2 und das Deckelteil 3 beide mit ihrer späteren Innenseite bezogen auf die Schwerkraftrichtung nach oben weisen. Das Einlegen des Wirkstoffapplikators 1 bzw. seines Kunststoffrohlings erfolgt im gezeigten Ausführungsbeispiel von Hand bei geschlossenem Abdeckelement 25. Es kann aber auch eine automatisierte Einlage bei geöffnetem Abdeckelement 25 vorgesehen sein.

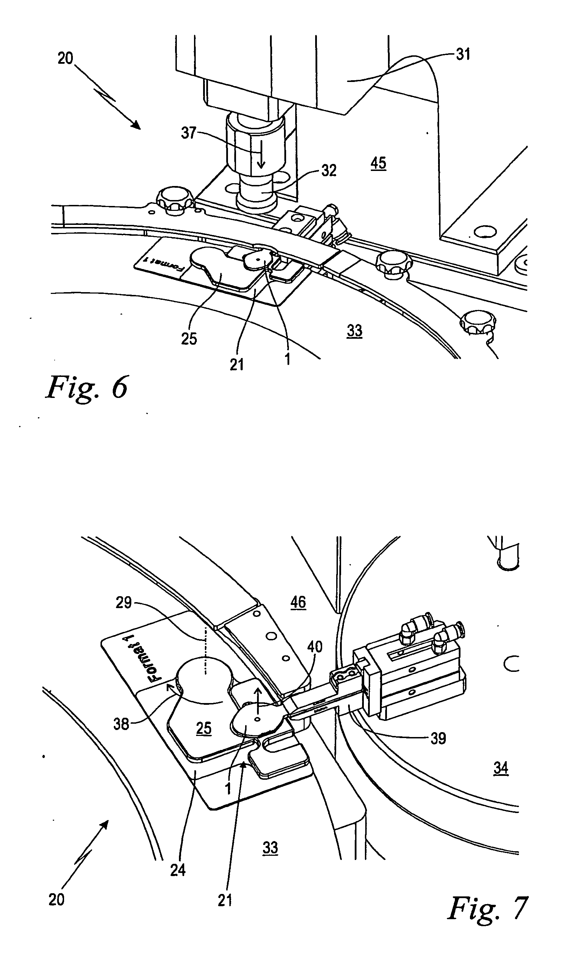

Fig. 6 zeigt in einer perspektivischen Detailansicht die Füll- und Verschließeinrichtung 20 nach Fig. 4 im Bereich ihrer fünften Station 45 mit der Siegelvorrichtung 31. Bei weiterhin geschlossenem Abdeckelement 25 ist der Wirkstoffapplikator 1 befüllt, bereits in der Konfiguration nach Fig. 3 provisorisch verschlossen und mittels des Aufnahmeelementes 21 auf dem ersten Rundlauftisch 33 unterhalb eines Siegelkopfes 32 der Siegelvorrichtung 31 positioniert. Der Siegelkopf 32 ist im gezeigten Ausführungsbeispiel ein Ultraschallsiegelkopf und wird zum Versiegeln entsprechend einem Pfeil 37 auf den provisorisch geschlossenen Wirkstoffapplikator 1 abgesenkt, woraufhin dann eine Ultraschallverschweißung des Deckelteils 3 mit dem Unterteil 2 (Fig. 1, 3) erfolgt.

Fig. 7 zeigt in einer perspektivischen Detailansicht die Füll- und Verschließeinrichtung 20 im Bereich ihrer sechsten Station 46 mit dem fertig verschweißten Wirkstoffapplikator 1 bei immer noch geschlossenem Abdeckelement 25. Ein auf dem zweiten Rundlauftisch 34 montierter Greifer 39 ergreift den Wirkstoffapplikator 1. Das Abdeckelement 25 ist gegenüber dem Basiselement 24 um eine in Gewichtskraftrichtung verlaufende Schwenkachse 29 schwenkbar und wird entsprechend einem Pfeil 38 aufgeschwenkt, wie dies weiter unten näher im Zusammenhang mit den Fig. 8 bis 11 beschrieben ist. Im aufgeschwenktem Zustand nach Fig. 11 wird der Wirkstoffapplikator 1 mittels des Greifers 39 entsprechend einem Pfeil 40 senkrecht nach oben angehoben und den nachfolgenden siebten, achten und neunten Stationen 47, 48, 49 entsprechend der Darstellung nach Fig. 4 zugeführt.

Fig. 8 zeigt in einer Längsschnittdarstellung das Aufnahmeelement 21 nach Fig. 4 mit dem eingelegten, noch offenen Rohling des Wirkstoffapplikators 1, wobei im gezeigten Längsschnitt nur das Unterteil 2 des Wirkstoffapplikators 1 mit der angeformten Applikationsröhre 7 erkennbar ist. Im Aufnahmeelement 21 ist eine Aufnahme 30 ausgeformt, in dem die Aufnahmeraumausformung 12 des Unterteils 2 derart zu liegen kommt, dass die Aufnahmeraumausformung 12 in Gewichtskraftrichtung nach oben offen und bereit zur Befüllung an der dritten Station 43 (Fig. 4) ist. Die Aufnahme 30 ist in ihrer Kontur an die Kontur der Aufnahmeraumausformung 12 derart angepasst, dass eine umlaufende Kante der Aufnahme 30 am Übergangsbereich zwischen der Aufnahmeraumausformung 12 und dem angrenzenden Siegelrand 5 anliegt und hierdurch eine Zentrierung des Unterteils 2 gegenüber dem Aufnahmeelement 21 in der horizontalen Richtung herbeiführt.

Darüber hinaus weist das Aufnahmeelement 21 eine geschlossen um die Aufnahme 30 umlaufende, bezogen auf die Gewichtskraftrichtung horizontal angeordnete Druckfläche 22 auf, die in ihrer geometrischen Ausgestaltung dem umlaufenden Siegelrand 5 bzw. der dort umlaufenden Stützfläche 9 (Fig. 1) entspricht. Das Unterteil 2 liegt mit seiner geschlossen umlaufenden Stützfläche 9 flächig auf der ebenfalls umlaufend geschlossenen Druckfläche 22 auf.

Im Aufnahmeelement 21 ist ein Aufnahmekanal 23 ausgeformt, der sich ausgehend von der Aufnahme 30 seitlich unter der Stützfläche 9 hindurch erstreckt, und der zwischen dem Basiselement 24 und dem Abdeckelement 25 ausgebildet ist. Hierzu ist im Basiselement 24 ein nach oben offener Kanal ausgebildet, der in seiner Form an die Form der Applikationsröhre 7 angepasst ist, und der nach oben durch das geschlossene Abdeckelement 25 verschlossen bzw. abgedeckt ist. Der verschlossene Aufnahmekanal 23 ist demnach durch die im Basiselement 24 ausgebildeten Kanalwände und nach oben durch das Abdeckelement 25 begrenzt und nimmt die Applikationsröhre 7 des Unterteils 2 auf.

Fig. 9 zeigt die vergrößerte Einzelheit IX nach Fig. 8 im Bereich des Abdeckelementes 25, des Siegelrandes 5 und der Applikationsröhre 7. Es ist erkennbar, dass die Applikationsröhre 7 im gezeigten Längsschnitt den Siegelrand 5 des Unterteils 2 bezogen auf dessen Ebene zumindest teilweise, hier vollständig derart übergreift, dass zwischen der Stützfläche 9 und der Applikationsröhre 7 bezogen auf die Vertikalrichtung eine Hinterschneidung gebildet ist. Im Bereich dieser Hinterschneidung weist die umlaufende Stützfläche 9 einen Stützflächenabschnitt 10 auf, der in der Vertikalrichtung nach unten durch die mit Abstand darunter sich erstreckende Applikationsröhre 7 zumindest teilweise, hier vollständig abgedeckt ist.

Aus der Zusammenschau der Fig. 8 und 9 wird deutlich, dass sich die umlaufende Druckfläche 22 sowohl über die Oberseite des Basiselementes 24 als auch über die Oberseite des Abdeckelementes 25 erstreckt. Dabei ist im Bereich des abgedeckten Stützflächenabschnitts 10 am Abdeckelement 25 auf seiner dem Aufnahmekanal 23 gegenüberliegenden Oberseite ein Druckflächenabschnitt 26 der Druckfläche 22 ausgebildet, auf dem der Stützflächenabschnitt 10 der Stützfläche 9 des Unterteils 2 aufliegt. Trotz der unterseitigen Abdeckung der Stützfläche 9 im Stützflächenabschnitt 10 durch die Applikationsröhre 7 erfährt die Stützfläche 9 insgesamt, also einschließlich ihres abgedeckten Stützflächenabschnittes 10 eine in Umfangsrichtung geschlossene vertikale Abstützung gegen Druck von oben, wie er beim Siegelvorgang nach den Fig. 6 und 10 auftritt.

Der Detailansicht nach Fig. 9 ist noch entnehmbar, dass am Wirkstoffapplikator 1 bezogen auf seinen durch das Unterteil 2 mit der Applikationsröhre 7 geführten Längsschnitt zwischen dem verdeckten Stützflächenabschnitt 10 und der Applikationsröhre 7 eine konkave Kante 11 ausgebildet ist. Das bewegliche Abdeckelement 25 ist mit einer in Richtung dieser konkaven Kante 11 weisenden, den Druckflächenabschnitt 26 nach innen zur Aufnahme 30 begrenzenden konvexen Andruckkante 27 versehen, an der das Unterteil 2 des Wirkstoffapplikators 1 mit seiner konkaven Kante 11 im eingelegten Zustand anliegt. Bezogen auf den gezeigten Längsschnitt geht die Andruckkante 27 des Abdeckelementes 25 ausgehend von dem oberen Druckflächenabschnitt 26 nach unten in eine dem Druckflächenabschnitt 26 in vertikaler Richtung gegenüberliegende Haltefläche 28 über, an der zumindest ein Abschnitt der Applikationsröhre 7 des Wirkstoffapplikators 1 im eingelegten Zustand anliegt. Insgesamt ragt damit das Abdeckelement 25 mit seiner Andruckkante 27, dem oberen Druckflächenabschnitt 26 und der unteren Haltefläche 28 in den Zwischenraum zwischen der Applikationsröhre 7 und dem Siegelrand 5, wodurch das Unterteil 2 vertikal nach oben, nach unten sowie horizontal seitlich gegenüber dem Aufnahmeelement 21 fixiert ist.

Fig. 10 zeigt die Anordnung nach Fig. 8, wobei das in Fig. 8 nicht dargestellte Deckelteil 3 entsprechend der Darstellung nach Fig. 3 auf das Unterteil 2 des Wirkstoffapplikators 1 aufgeklappt wurde, nachdem die zuvor nach oben offene Aufnahmeraumausformung 12 in der Konfiguration nach Fig. 8 in der dritten Station 43 (Fig. 4) mit dem Wirkstoffpräparat befüllt wurde. In der fünften Station 45 (Fig. 4, 6) wird nun entsprechend der Darstellung nach Fig. 10 der Siegelkopf 32 entsprechend dem Pfeil 37 abgesenkt und auf den oberen Siegelrand 6 des oben aufliegenden Deckelteils 3 gepresst. Der das Gleichgewicht haltende Gegendruck zum in Richtung des Pfeiles 37 wirkenden Pressdruck wird durch die umlaufende Druckfläche 22 an der ebenfalls umlaufenden Stützfläche 9 einschließlich des Druckflächenabschnittes 26 am verdeckten Stützflächenabschnitt 10 erzeugt. Der Siegelkopf 32 weist eine umlaufende Siegelkante auf, deren Kontur der umlaufenden Kontur der Siegelränder 5, 6 entspricht, so dass hier unter Bildung des befüllten und hermetisch verschlossenen Aufnahmeraumes 4 eine Ultraschallverschweißung des Deckelteils 3 mit dem Unterteil 2 erfolgt.

Nach dem Versiegeln bzw. nach dem Ultraschallverschweißen wird das Aufnahmeelement 21 mit dem darin gehaltenen Wirkstoffapplikator 1 entsprechend der Darstellung nach den Fig. 4, 7 mittels des ersten Rundlauftisches 33 zur sechsten Station 46 verfahren, wo die Entnahme des Wirkstoffapplikators 1 aus dem Aufnahmeelement 41 mittels des Greifers 39 erfolgen soll. Diese Situation ist in Fig. 11 detailliert dargestellt, wobei die Anordnung nach Fig. 8 mit gegenüber dem Basiselement 24 des Aufnahmeelementes 21 aufgeschwenktem Abdeckelement 25 gezeigt ist. Es ist erkennbar, dass die vertikale Schwenkachse 29 senkrecht auf der umlaufenden Druckfläche 22 steht. Insbesondere aus der Zusammenschau der Fig. 9 und 11 wird deutlich, dass durch die senkrechte Lage der Schwenkachse 29 das Abdeckelement 25 aus dem Hinterschneidungsbereich zwischen dem abgedeckten Stützflächenabschnitt 10 und der Applikationsröhre 7 des Wirkstoffapplikators 1 herausgeschwenkt werden kann, ohne dass dies eine Lageänderung bzw. ein Anheben des Wirkstoffapplikators 1 zur Folge hat. Entsprechend der Darstellung nach Fig. 11 ist das Abdeckelement 25 so weit aufgeschwenkt, dass der Aufnahmekanal 23 bezogen auf die horizontal projizierte Länge der Applikationsröhre 7 vollständig nach oben offen ist. Dies erlaubt es, wie aus der Zusammenschau der Fig. 7 und 11 ersichtlich, den fertig befüllten und versiegelten Wirkstoffapplikator 1 mittels des Greifers 39 automatisiert nach oben in Richtung des Pfeiles 40 anzuheben und vollständig aus dem Aufnahemeelement 21 herauszunehmen, ohne dass die Applikationsröhre 7 mit dem Abdeckelement 25 kollidiert.

Eingangs wurde erwähnt, dass der geöffnete Rohling des Wirkstoffapplikators 1 entsprechend der Darstellung nach Fig. 5 manuell in das Aufnahmeelement 21 bei geschlossenem Abdeckelement 25 eingelegt wird. Dies erfolgt, indem der Rohling des Wirkstoffapplikators 1 entsprechend der Darstellung nach Fig. 8 zumindest näherungsweise in Längsrichtung der Applikationsröhre 7 manuell in den Aufnahmekanal 23 eingeschoben und dann mit der Aufnahmeraumausformung 12 in die Aufnahme 30 eingedrückt wird. Dieser komplexe Bewegungsvorgang erfordert eine manuelle Handhabung. Es kann aber auch analog zur Darstellung zu Fig. 11 ein automatisiertes Einlegen des Applikatorrohlings entgegen dem Pfeil 40 von oben nach unten mittels eines geeigneten Greifers oder dergleichen zweckmäßig sein, wobei dann das Abdeckelement 25 zunächst entsprechend der Darstellung nach Fig. 11 geöffnet ist und erst anschließend in die Position 8, 9 verschwenkt wird.

## Patentansprüche

1. Füll- und Verschließeinrichtung (20) für einen Wirkstoffapplikator (1), wobei der Wirkstoffapplikator (1) ein Unterteil (2) und ein Deckelteil (3) aufweist, zwischen denen ein Aufnahmeraum (4) für ein Wirkstoffpräparat ausgebildet ist, wobei das Unterteil (2) und das Deckelteil (3) je einen umlaufenden Siegelrand (5, 6) zur dichtenden Verbindung des Deckelteils (3) mit dem Unterteil (2) aufweisen, wobei das Unterteil (2) mit einer Applikationsröhre (7) versehen ist, die in den Aufnahmeraum (4) mittels einer inneren Mündung (8) mündet, wobei der Siegelrand (5) des Unterteils (2) auf seiner dem Deckelteil (3) abgewandten Unterseite eine umlaufende Stützfläche (9) bildet, wobei die Applikationsröhre (7) den Siegelrand (5) bezogen auf seine Ebene zumindest teilweise derart übergreift, dass die umlaufende Stützfläche (9) einen durch die Applikationsröhre (7) zumindest teilweise verdeckten Stützflächenabschnitt (10) aufweist, wobei die Füll- und Verschließeinrichtung (20) ein Aufnahmeelement (21) für das Unterteil (2) des Wirkstoffapplikators (1) mit einer umlaufenden Druckfläche (22) zur Anlage der Stützfläche (9) des Unterteils (2) sowie mit einem unter die Druckfläche (22) reichenden Aufnahmekanal (23) für die Applikationsröhre (7) des Unterteils (2) aufweist, wobei das Aufnahmeelement (21) mindestens zweiteilig mit einem Basiselement (24) und einem gegenüber dem Basiselement (24) beweglichen Abdeckelement (25) aufgebaut ist, wobei der Aufnahmekanal (23) zwischen dem Basiselement (24) und dem Abdeckelement (25) ausgebildet ist, wobei sich die umlaufende Druckfläche (22) über das Basiselement (24) und das Abdeckelement (25) derart erstreckt, dass auf der dem Aufnahmekanal (23) gegenüberliegenden Oberseite des Abdeckelementes (25) ein Druckflächenabschnitt (26) zur Anlage des verdeckten Stützflächenabschnittes (10) des Wirkstoffapplikators (1) ausgebildet ist.

2. Füll- und Verschließeinrichtung nach Anspruch 1,
**dadurch gekennzeichnet, dass** am Wirkstoffapplikator (1) bezogen auf seinen Längsschnitt zwischen dem verdeckten Stützflächenabschnitt (10) und der Applikationsröhre (7) eine konkave Kante (11) ausgebildet ist, und dass am Abdeckelement (25) eine Andruckkante (27) zur Anlage an der konkaven Kante (11) des Wirkstoffapplikators (1) ausgebildet ist.

3. Füll- und Verschließeinrichtung nach Anspruch 2,
**dadurch gekennzeichnet, dass** die Andruckkante (27) des Abdeckelementes (25) in eine Haltefläche (28) zur Anlage an zumindest einem Abschnitt der Applikationsröhre (7) des Wirkstoffapplikators (1) übergeht.

4. Füll- und Verschließeinrichtung nach einem der Ansprüche 1 bis 3,
**dadurch gekennzeichnet, dass** im Basiselement (24) innerhalb der umlaufenden Druckfläche (22) eine Aufnahme (30) für eine Aufnahmeraumausformung (12) des Unterteils (2) des Wirkstoffapplikators (1) ausgebildet ist.

5. Füll- und Verschließeinrichtung nach einem der Ansprüche 1 bis 4,
**dadurch gekennzeichnet, dass** das Abdeckelement (25) gegenüber dem Basiselement (24) um eine Schwenkachse (29) schwenkbar ist, wobei die Schwenkachse (29) senkrecht auf der umlaufenden Druckfläche (22) steht.

6. Füll- und Verschließeinrichtung nach einem der Ansprüche 1 bis 5,
**dadurch gekennzeichnet, dass** die Füll- und Verschließeinrichtung eine Siegelvorrichtung (31) insbesondere in Form einer Ultraschallschweißvorrichtung mit einem in seiner Form mit den umlaufenden Siegelrändern (5, 6) und mit der umlaufenden Druckfläche (22) korrespondierenden Siegelkopf (32) aufweist, wobei der Siegelkopf (32) unter Zwischenlage der Siegelränder (5, 6) auf die Druckfläche (22) des Aufnahmeelementes (21) absenkbar ist.

7. Verfahren zum Füllen und Verschließen eines Wirkstoffapplikators (1) mittels einer Füll- und Verschließeinrichtung (20) nach einem der Ansprüche 1 bis 6, umfassend folgende Verfahrensschritte:
- Das Unterteil (2) des Wirkstoffapplikators (1) wird derart in das Aufnahmeelement (21) der Füll- und Verschließeinrichtung (20) eingelegt, dass die Applikationsröhre (7) in den Aufnahmekanal (23) hineinragt, wobei die umlaufende Stützfläche (9) auf der umlaufenden Druckfläche (22) und der verdeckte Stützflächenabschnitt (10) auf dem Druckflächenabschnitt (26) des Abdeckelementes (25) aufliegt;
- eine Aufnahmeraumausformung (12) des Unterteils (2) des Wirkstoffapplikators (1) wird mit dem Wirkstoffpräparat befüllt;
- das Oberteil (3) des Wirkstoffapplikators (1) wird lagerichtig derart auf das Unterteil (2) gebracht, dass die umlaufenden Siegelränder (5, 6) aufeinander liegen;
- das Oberteil (3) des Wirkstoffapplikators (1) wird auf das Unterteil (2) an den umlaufenden Siegelrändern (5, 6) unter Gegendruck der umlaufenden Druckfläche (22) aufgesiegelt;
- das Abdeckelement (25) wird gegenüber dem Basiselement (24) derart bewegt, dass der zwischen ihnen ausgebildete Aufnahmekanal (23) freiliegt;
- der fertig befüllte und versiegelte Wirkstoffapplikator (1) wird aus dem Aufnahmeelement (21) entnommen.

8. Verfahren nach Anspruch 7,
**dadurch gekennzeichnet, dass** das Abdeckelement (25) nach dem Siegelvorgang gegenüber dem Basiselement (24) um eine senkrecht auf der umlaufenden Druckfläche (22) stehende Schwenkachse (29) geschwenkt wird, und dass der fertig befüllte und versiegelte Wirkstoffapplikator (1) mittels einer senkrechten Hubbewegung aus dem Aufnahmeelement (21) entnommen wird.

## Claims

1. Filling and closing device (20) for an agent applicator (1), wherein the agent applicator (1) has a lower part (2) and a cover part (3), between which a receiving area (4) for an agent preparation is formed, wherein the lower part (2) and the cover part (3) each have a surrounding sealing edge (5, 6) for sealed connection of the cover part (3) to the lower part (2), wherein the lower part (2) is provided with an application tube (7) which opens into the receiving area (4) by means of an inner opening area (8), wherein the sealing edge (5) of the lower part (2) forms on its lower side facing away from the cover part (3) a surrounding support area (9), wherein the applicator tube (7) engages over the sealing edge (5) at least partially in relation to the plane thereof in such a way that the surrounding support area (9) has a support area portion (10) at least partially concealed by the application tube (7), wherein the filling and closing device (20) has a receiving element (21) for the lower part (2) of the agent applicator (1) with a surrounding pressure area (22) for abutment of the support area (9) of the lower part (2) and with a receiving channel (23), extending below the pressure area (22), for the application tube (7) of the lower part (2), wherein the receiving element (21) is constructed in at least two parts with a base element (24) and a cover element (25) which is movable with respect to the base element (24), wherein the receiving channel (23) is formed between the base element (24) and the cover element (25), wherein the surrounding pressure area (22) extends over the base element (24) and the cover element (25) in such a way that on the upper side of the cover element (25) lying opposite the receiving channel (23) a pressure area portion (26) is formed for abutment of the concealed support area portion (10) of the agent applicator (1).

2. Filling and closing device according to claim 1,
**characterised in that** a concave edge (11) is formed on the agent applicator (1) with respect to its longitudinal section between the concealed support area portion (10) and the application tube (7), and a pressure edge (27) is formed on the cover element (25) for abutment against the concave edge (11) of the agent applicator (1).

3. Filling and closing device according to claim 2,
**characterised in that** the pressure edge (27) of the cover element (25) transforms into a holding area (28) for abutment against at least one portion of the application tube (7) of the agent applicator (1).

4. Filling and closing device according to one of the claims 1 to 3,
**characterised in that** a receiving element (30) for a receiving area formation (12) of the lower part (2) of the agent applicator (1) is formed in the base element (24) within the surrounding pressure area (22).

5. Filling and closing device according to one of the claims 1 to 4,
**characterised in that** the cover element (25) can be pivoted about a pivot axis (29) with respect to the base element (24), wherein the pivot axis (29) is perpendicular with respect to the surrounding pressure area (22).

6. Filling and closing device according to one of the claims 1 to 5,
**characterised in that** the filling and closing device has a sealing unit (31) in particular in the form of an ultrasonic welding unit with a sealing head (32) corresponding in its shape to the surrounding sealing edges (5, 6) and to the surrounding pressure area (22), wherein the sealing head (32) can be lowered, with the interposition of the sealing edges (5, 6), onto the pressure area (22) of the receiving element (21).

7. Method for filling and closing an agent applicator (1) by means of a filling and closing device (20) according to one of the claims 1 to 6, comprising the following method steps:
- the lower part (2) of the agent applicator (1) is laid in the receiving element (21) of the filling and closing device (20) in such a way that the application tube (7) projects into the receiving channel (23), wherein the surrounding support area (9) lies on the surrounding pressure area (22), and the concealed support area portion (10) lies on the pressure area portion (26) of the cover element (25);
- a receiving area formation (12) of the lower part (2) of the agent applicator (1) is filled with the agent preparation;
- the upper part (3) of the agent applicator (1) is brought into the correct position on the lower part (2) in such a way that the surrounding sealing edges (5, 6) lie one on top of the other;
- the upper part (3) of the agent applicator (1) is sealed onto the lower part (2) at the surrounding sealing edges (5, 6) with counter-pressure of the surrounding pressure area (22);
- the cover element (25) is moved with respect to the base element (24) in such a way that the receiving channel (23) formed between them is exposed;
- the completely filled and sealed agent applicator (1) is removed from the receiving element (21).

8. Method according to claim 7,
**characterised in that** the cover element (25) is pivoted after the sealing process with respect to the base element (24) about a pivot axis (29) lying perpendicularly with respect to the surrounding pressure area (22), and the completely filled and sealed agent applicator (1) is removed by means of vertical lifting movement from the receiving element (21).

## Revendications

1. Dispositif de remplissage et de fermeture (20) pour un applicateur de matière active (1), étant précisé que l'applicateur de matière active (1) comporte une partie inférieure (2) et une partie formant couvercle (3) entre lesquelles est formé un logement (4) pour une préparation de matière active, étant précisé que la partie inférieure (2) et la partie formant couvercle (3) présentent chacune un bord de scellage périphérique (5, 6) pour une liaison étanche de la partie formant couvercle (3) avec la partie inférieure (2), que la partie inférieure (2) est pourvue d'un tube d'application (7) qui débouche dans le logement (4) grâce à une ouverture intérieure (8), que le bord de scellage (5) de la partie inférieure (2) forme sur son côté inférieur opposé à la partie formant couvercle (3) une surface d'appui périphérique (9), que le tube d'application (7) recouvre au moins en partie le bord de scellage (5) par rapport au plan de celui-ci de telle sorte que la surface d'appui périphérique (9) présente une section de surface d'appui (10) au moins en partie couverte par le tube d'application (7), que le dispositif de remplissage et de fermeture (20) comporte un élément de réception (21) pour la partie inférieure (2) de l'applicateur de matière active (1), avec une surface de pression périphérique (22) pour l'application de la surface d'appui (9) de la partie inférieure (2) et avec un conduit de réception (23), qui s'étend jusque sous la surface de pression (22), pour le tube d'application (7) de la partie inférieure (2), que l'élément de réception (21) est construit au moins en deux parties, avec un élément de base (24) et un élément de recouvrement (25) mobile par rapport à l'élément de base (24), que le conduit de réception (23) est formé entre l'élément de base (24) et l'élément de recouvrement (25), et que la surface de pression périphérique (22) s'étend sur l'élément de base (24) et l'élément de recouvrement (25) de telle sorte que sur le côté supérieur de l'élément de recouvrement (25) opposé au conduit de réception (23) est formée une section de surface de pression (26) pour l'application de la section de surface d'appui couverte (10) de l'applicateur de matière active (1).

2. Dispositif de remplissage et de fermeture selon la revendication 1,
**caractérisé en ce qu'**il est prévu sur l'applicateur de matière active (1) par rapport à sa coupe longitudinale entre la section de surface d'appui couverte (10) et le tube d'application (7) un bord concave (11), et **en ce que** sur l'élément de recouvrement (25) est formé un bord de pression (27) pour l'application contre le bord concave (11) de l'applicateur de matière active (1).

3. Dispositif de remplissage et de fermeture selon la revendication 2,
**caractérisé en ce que** le bord de pression (27) de l'élément de recouvrement (25) se prolonge par une surface de retenue (28) pour l'application contre au moins une section du tube d'application (7) de l'applicateur de matière active (1).

4. Dispositif de remplissage et de fermeture selon l'une des revendications 1 à 3,
**caractérisé en ce qu'**il est prévu dans l'élément de base (24), à l'intérieur de la surface de pression périphérique (22), un logement (30) pour une protubérance de logement (12) de la partie inférieure (2) de l'applicateur de matière active (1).

5. Dispositif de remplissage et de fermeture selon l'une des revendications 1 à 4,
**caractérisé en ce que** l'élément de recouvrement (25) est apte à pivoter sur un axe de pivotement (29) par rapport à l'élément de base (24), étant précisé que l'axe de pivotement (29) se trouve à la verticale sur la surface de pression périphérique (22).

6. Dispositif de remplissage et de fermeture selon l'une des revendications 1 à 5,
**caractérisé en ce que** le dispositif de remplissage et de fermeture présente un dispositif de scellage (31) en particulier sous la forme d'un dispositif de soudage par ultrasons, avec une tête de scellage (32) qui correspond par sa forme aux bords de scellage périphériques (5, 6) et à la surface de pression périphérique (22), étant précisé que la tête de scellage (32) est apte à être abaissée sur la surface de pression (22) de l'élément de réception (21), avec les bords de scellage (5, 6) entre les deux.

7. Procédé pour remplir et fermer un applicateur de matière active (1) à l'aide d'un dispositif de remplissage et de fermeture (20) selon l'une des revendications 1 à 6, comprenant les étapes suivantes :
- la partie inférieure (2) de l'applicateur de matière active (1) est placée dans l'élément de logement (21) du dispositif de remplissage et de fermeture (20) de telle sorte que le tube d'application (7) dépasse dans le conduit de logement (23), étant précisé que la surface d'appui périphérique (9) est posée sur la surface de pression périphérique (22), et la section de surface d'appui couverte (10) sur la section de surface de pression (26) de l'élément de recouvrement (25) ;
- une protubérance de logement (12) de la partie inférieure (2) de l'applicateur de matière active (1) est remplie avec la préparation de matière active ;
- la partie supérieure (3) de l'applicateur de matière active (1) est amenée, dans la bonne position, sur la partie inférieure (2) de telle sorte que les bords de scellage périphériques (5, 6) soient posés l'un sur l'autre ;
- la partie supérieure (3) de l'applicateur de matière active (1) est scellée sur la partie inférieure (2) au niveau des bords de scellage périphériques (5, 6), sous la contre-pression de la surface de pression périphérique (22) ;
- l'élément de recouvrement (25) est déplacé par rapport à l'élément de base (24) de telle sorte que le conduit de logement de réception (23) formé entre les deux soit dégagé ;
- l'applicateur de matière active (1), une fois rempli et scellé, est enlevé de l'élément de réception (21).

8. Procédé selon la revendication 7,
**caractérisé en ce que** l'élément de recouvrement (25), après l'opération de scellage, pivote par rapport à l'élément de base (24) sur un axe de pivotement (29) qui se trouve à la verticale sur la surface de pression périphérique (22), et **en ce que** l'applicateur de matière active (1), une fois rempli et scellé, est enlevé de l'élément de réception (21) grâce à un mouvement de soulèvement vertical.
